(19) Europäisches Patentamt
European Patent Office
Office européen des brevets

(11) Veröffentlichungsnummer: **0 091 638**
**A1**

# (12) EUROPÄISCHE PATENTANMELDUNG

(21) Anmeldenummer: 83103303.0

(22) Anmeldetag: 05.04.83

(51) Int. Cl.³: C 07 C 127/19
A 01 N 47/30

(30) Priorität: 10.04.82 DE 3213375

(43) Veröffentlichungstag der Anmeldung: 19.10.83 Patentblatt 83/42

(84) Benannte Vertragsstaaten: DE FR GB

(71) Anmelder: BASF Aktiengesellschaft
Carl-Bosch-Strasse 38
D-6700 Ludwigshafen(DE)

(72) Erfinder: Koenig, Karl-Heinz, Dr.
Pierstrasse 8 a
D-6710 Frankenthal(DE)

(72) Erfinder: Schwendemann, Volker, Dr.
Hauptstrasse 64 a
D-6901 Wiesenbach(DE)

(72) Erfinder: Schirmer, Ulrich, Dr.
Berghalde 79
D-6900 Heidelberg(DE)

(72) Erfinder: Liesner, Michael, Dr.
Haardtstrasse 6
D-6714 Weisenheim(DE)

(72) Erfinder: Wuerzer, Bruno, Dr. Dipl.-Landwirt
Ruedigerstrasse 13
D-6701 Otterstadt(DE)

## (54) Halogenalkyl-phenylharnstoffe, Verfahren zu ihrer Herstellung und ihre Verwendung zur Bekämpfung unerwünschten Pflanzenwuchses.

(57) Die Erfindung betrifft Halogenalkyl-phenylharnstoffe der Formel

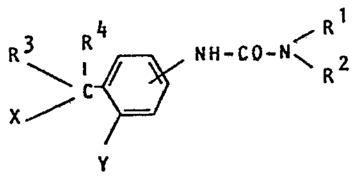

in der
$R^1$, $R^2$, $R^3$, $R^4$, X und Y die in der Beschreibung genannten Bedeutungen haben,
Verfahren zu ihrer Herstellung und ihre Verwendung zur Bekämpfung unerwünschten Pflanzenwuchses.

EP 0 091 638 A1

# Halogenalkyl-phenylharnstoffe, Verfahren zu ihrer Herstellung und ihre Verwendung zur Bekämpfung unerwünschten Pflanzenwuchses

Die Erfindung betrifft Halogenalkyl-phenylharnstoffe, Verfahren zu ihrer Herstellung, Herbizide, die diese Verbindungen als Wirkstoffe enthalten, sowie ein Verfahren zur Bekämpfung unerwünschten Pflanzenwuchses mit diesen Wirkstoffen.

Es ist bekannt, daß 4-Alkylphenylharnstoffe herbizide Eigenschaften haben (US-PS 2 655 447).

Es wurde gefunden, daß Halogenalkyl-phenylharnstoffe der Formel

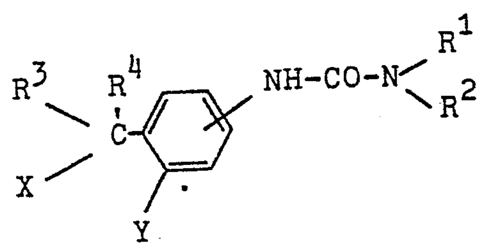 (I),

in der

$R^1$ und $R^2$ jeweils unabhängig voneinander Wasserstoff, Methoxy oder Methyl bedeuten,

Y Wasserstoff oder Chlor,

X Chlor oder Brom und

$R^3$ und $R^4$ jeweils unabhängig voneinander Wasserstoff oder Alkyl bedeuten,

mit der Maßgabe, daß die Gruppe $-NH-CO-NR^1R^2$ nicht in o-Stellung zu Y oder zum Rest $-CXR^3R^4$ steht, eine herbizide Wirkung bei zahlreichen unerwünschten Pflanzen haben.

H/Kl

Die Halogenalkyl-phenylharnstoffe der Formel

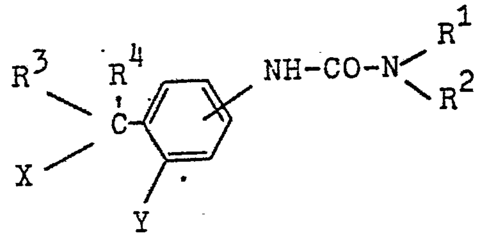

(I),

in der

$R^1$ und $R^2$ jeweils unabhängig voneinander Wasserstoff, Methoxy oder Methyl bedeuten,

Y Wasserstoff oder Chlor,

X Chlor oder Brom und

$R^3$ und $R^4$ jeweils unabhängig voneinander Wasserstoff oder Alkyl bedeuten,

mit der Maßgabe, daß $R^3$ und $R^4$ nicht gleichzeitig Wasserstoff bedeuten, wenn $R^1$ und $R^2$ für Methyl und Y für Wasserstoff stehen, und daß die Gruppe $-NH-CO-NR^1R^2$ nicht in o-Stellung zu Y oder zum Rest $-CXR^3R^4$ steht, sind neu.

Die Halogenalkyl-phenylharnstoffe der Formel

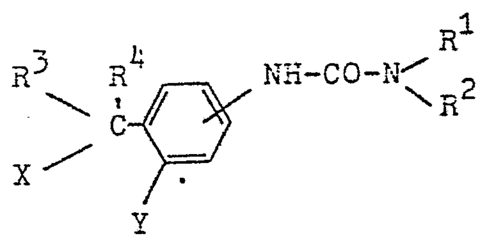

(I),

in der

$R^1$ und $R^2$ jeweils unabhängig voneinander Wasserstoff, Methoxy oder Methyl bedeuten,

Y Wasserstoff oder Chlor,

X Chlor oder Brom und

$R^3$ und $R^4$ jeweils unabhängig voneinander Wasserstoff oder Alkyl bedeuten,

mit der Maßgabe, daß $R^3$ und $R^4$ nicht gleichzeitig Wasserstoff bedeuten, wenn $R^1$ und $R^2$ für Methyl und Y für Wasserstoff stehen, und daß die Gruppe $-NH-CO-NR^1R^2$ nicht in o-Stellung zu Y oder zum Rest $-CXR^3R^4$ steht, können in an sich bekannter Weise durch Umsetzung von Halogenalkyl-phenylisocyanaten der Formel

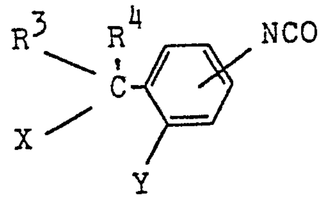

(II),

in der
$R^3$, $R^4$, X und Y die oben genannten Bedeutungen haben, oder von N-(Halogenalkyl-phenyl)-carbamoylhalogeniden der Formel

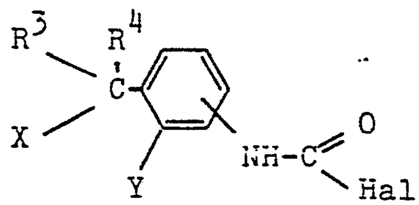

(III),

in der
$R^3$, $R^4$, X und Y die oben genannten Bedeutungen haben und Hal für Halogen steht,
in an sich üblicher Weise mit einem Amin der Formel

$$HNR^1R^2 \qquad (IV),$$

in der
$R^1$ und $R^2$ die oben genannten Bedeutungen haben, erhalten werden (Houben-Weyl, Methoden der organischen Chemie, Bd. VIII, S. 132 ff, Georg Thieme-Verlag, Stuttgart, 4. Auflage, 1952).

Überraschenderweise reagiert dabei das Halogenalkyl-phenylisocyanat bzw. das ihm entsprechende Carbamoylchlorid mit dem Amin nur an der Isocyanatfunktion. Die als Nebenreaktion zu erwartende Umsetzung des Amins mit der alphaHalogenalkylfunktion läuft nicht ab.

Die Umsetzung der Halogenalkyl-phenylisocyanate der Formel II mit den Aminen der Formel III kann in Gegenwart eines für Reaktionen mit Isocyanaten üblichen Katalysators, z.B. eines tertiären Amins, wie Triethylamin, 1,4-Diazabicyclo[2,2,2]-octan, stickstoffhaltiger Heterocyclen, wie Pyridin, 1,2-Dimethylimidazol oder organischer Zinnverbindungen, wie Dibutylzinndiacetat, Dimethylzinndichlorid durchgeführt werden.

Zweckmäßigerweise wird die Umsetzung in Gegenwart eines inerten organischen Lösungsmittels durchgeführt. Geeignet sind Kohlenwasserstoffe, wie Ligroin, Benzin, Toluol, Pentan, Cyclohexan, Halogenkohlenwaserstoffe, wie Methylenchlorid, Chloroform, Dichlorethan, Chlorbenzol, o-, m- oder p-Dichlorbenzol, Nitrokohlenwasserstoffe, wie Nitrobenzol, Nitromethan, Nitrile, wie Acetonitril, Butyronitril, Benzonitril, Ether, wie Diethylether, Tetrahydrofuran, Dioxan, Ester, wie Essigsäuremethylester, Propionsäuremethylester, Ketone, wie Aceton, Methylethylketon, oder Amide, wie Dimethylformamid, Formamid.

Die Umsetzung wird bei einer Temperatur im Bereich zwischen -20 und +100°C, vorzugsweise zwischen 0 und +60°C, durchgeführt.

Die folgenden Beispiele erläuten die Herstellung der Halogenalkylphenylharnstoffe der Formel I.

Beispiel 1

66 Gewichtsteile p-(1-Chlorethyl)-phenylisocyanat in 200 Gewichtsteilen Essigester wurden bei 15°C mit 15 Gewichtsteilen Dimethylamin in 100 Teilen Essigester versetzt und 1 Stunde lang zur Reaktion gebracht. Der entstandene Niederschlag wurde abgesaugt; es wurden 77 Gewichtsteile N-[4-(1-Chlorethyl)-phenyl]-N',N'-dimethylharnstoff vom Schmelzpunkt 120°C erhalten.

Beispiel 2

8 Gewichtsteile N-Methoxy-N-methylamin in 50 Gewichteilen Toluol wurden bei 0°C mit 23,5 Gewichtsteilen 4-(1-Chlorethyl)-phenylisocyanat in 100 Gewichtsteilen Toluol 30 Minuten lang zur Reaktion gebracht. Der entstandene Niederschlag wurde abgesaugt; es wurden 12 Gewichtsteile N-[4-(1-Chlorethyl)-phenyl]-N'-methoxy-N'-methylharnstoff vom Schmelzpunkt 86°C erhalten.

Beispiel 3

19,5 Gewichtsteile N-Methoxy-N-methylamin in 100 Gewichtsteilen Toluol wurden bei 40°C mit 48 Gewichtsteilen 4-(Chlormethyl)-phenylisocyanat in 100 Gewichtsteilen Toluol 10 Minuten lang zur Reaktion gebracht. Der entstandene Niederschlag wurde abgesaugt; es wurden 29,5 Gewichtsteile N-(4-Chlormethylphenyl)-N'-methoxy-N'-methylharnstoff vom Schmelzpunkt 78°C erhalten.

Beispiel 4

3,1 Gewichtsteile Methylamin in 50 Gewichtsteilen Toluol werden bei 0°C in 16,7 Gewichtsteilen 4-Chlormethylphenylisocyanat in 150 Gewichtsteilen Essigester zugetropft und 1 Stunde lang bei 10°C nachgerührt. Nach Absaugen des Niederschlages wurden 18 Gewichtsteile N-(4-Chlormethyl-phenyl)-N'-methylharnstoff vom Schmelzpunkt 128°C erhalten.

Folgende Verbindungen der Formel I können beispielsweise erhalten werden:

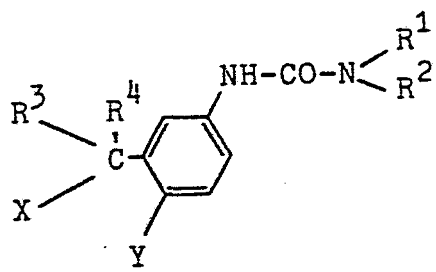

| Nr. | $R^1$ | $R^2$ | $R^3$ | $R^4$ | X | Y | physik. Daten |
|---|---|---|---|---|---|---|---|
| 5 | $CH_3$ | $CH_3$ | H | $CH_3$ | Cl | H | Fp. 128-130°C |
| 6 | $CH_3$ | H | H | $CH_3$ | Cl | H | |
| 7 | $CH_3$ | $OCH_3$ | H | $CH_3$ | Cl | H | |
| 8 | $CH_3$ | $CH_3$ | H | $C_2H_5$ | Cl | H | |
| 9 | CH | H | H | $C_2H_5$ | Cl | H | |
| 10 | $CH_3$ | $OCH_3$ | H | $C_2H_5$ | Cl | H | |
| 11 | $CH_3$ | $CH_3$ | $CH_3$ | $CH_3$ | Cl | H | |
| 12 | $CH_3$ | H | $CH_3$ | $CH_3$ | Cl | H | |
| 13 | $CH_3$ | $OCH_3$ | $CH_3$ | $CH_3$ | Cl | H | |
| 14 | $CH_3$ | $CH_3$ | H | $CH_3$ | Br | H | |
| 15 | $CH_3$ | $OCH_3$ | H | $CH_3$ | Br | H | Fp. 72-74 |
| 16 | $CH_3$ | $CH_3$ | $CH_3$ | $CH_3$ | Br | H | |
| 17 | $CH_3$ | $OCH_3$ | $CH_3$ | $CH_3$ | Br | H | |
| 49 | $CH_3$ | $OCH_3$ | H | H | Br | H | Fp. 113-114 |
| 50 | $CH_3$ | $CH_3$ | H | H | Br | H | Fp. 148-150 |

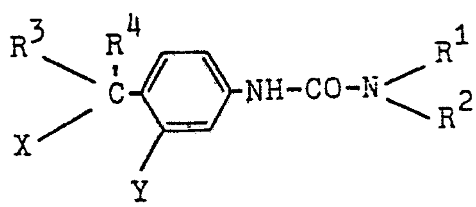

| Nr. | $R^1$ | $R^2$ | $R^3$ | $R^4$ | X | Y | physik. Daten |
|---|---|---|---|---|---|---|---|
| 18 | $CH_3$ | $CH_3$ | H | $CH_3$ | Cl | H | |
| 19 | $CH_3$ | H | H | $CH_3$ | Cl | H | |
| 20 | $CH_3$ | $OCH_3$ | H | $CH_3$ | Cl | H | |
| 21 | $CH_3$ | $CH_3$ | H | $C_2H_5$ | Cl | H | |

| Nr. | $R^1$ | $R^2$ | $R^3$ | $R^4$ | X | Y | physik. Daten |
|---|---|---|---|---|---|---|---|
| 22 | $CH_3$ | H | H | $C_2H_5$ | Cl | H | |
| 23 | $CH_3$ | $OCH_3$ | H | $C_2H_5$ | Cl | H | |
| 24 | $CH_3$ | $CH_3$ | $CH_3$ | $CH_3$ | Cl | H | |
| 25 | $CH_3$ | H | $CH_3$ | $CH_3$ | Cl | H | |
| 26 | $CH_3$ | $OCH_3$ | $CH_3$ | $CH_3$ | Cl | H | |
| 27 | $CH_3$ | $CH_3$ | H | $CH_3$ | Br | H | |
| 28 | $CH_3$ | $OCH_3$ | H | $CH_3$ | Br | H | |
| 29 | $CH_3$ | $CH_3$ | $CH_3$ | $CH_3$ | Br | H | |
| 30 | $CH_3$ | $OCH_3$ | $CH_3$ | $CH_3$ | Br | H | |
| 31 | $CH_3$ | $CH_3$ | H | $CH_3$ | Cl | Cl | |
| 32 | $CH_3$ | H | H | $CH_3$ | Cl | Cl | |
| 33 | $CH_3$ | $OCH_3$ | H | $CH_3$ | Cl | Cl | |
| 34 | $CH_3$ | $CH_3$ | H | $C_2H_5$ | Cl | Cl | |
| 35 | $CH_3$ | H | H | $C_2H_5$ | Cl | Cl | |
| 36 | $CH_3$ | $OCH_3$ | H | $C_2H_5$ | Cl | Cl | |
| 37 | $CH_3$ | $CH_3$ | $CH_3$ | $CH_3$ | Cl | Cl | |
| 38 | $CH_3$ | H | $CH_3$ | $CH_3$ | Cl | Cl | |
| 39 | $CH_3$ | $OCH_3$ | $CH_3$ | $CH_3$ | Cl | Cl | |
| 40 | $CH_3$ | $CH_3$ | H | $CH_3$ | Br | Cl | |
| 41 | $CH_3$ | $OCH_3$ | H | $CH_3$ | Br | Cl | |
| 42 | $CH_3$ | $CH_3$ | $CH_3$ | $CH_3$ | Br | Cl | |
| 43 | $CH_3$ | $OCH_3$ | $CH_3$ | $CH_3$ | Br | Cl | |
| 44 | $CH_3$ | $OCH_3$ | H | H | Br | H | Fp. 102-104°C |
| 45 | $CH_3$ | $OCH_3$ | H | H | Br | Cl | Fp. 111-113 |
| 46 | $CH_3$ | $CH_3$ | H | H | Br | Cl | Fp. 130-132 |
| 47 | $CH_3$ | $CH_3$ | H | H | Cl | H | Fp. 110-114 |
| 48 | $CH_3$ | $CH_3$ | H | H | Br | H | Fp. 76-78 |

Die Halogenalkyl-phenylharnstoffe der Formel I sind durch die in alpha-Position zum Phenylring ständigen Halogenatome außerordentlich reaktionsfähig und deshalb auch als Zwischenprodukte für chemische Synthesen geeignet. Durch

Umsetzung der erfindungsgemäßen Harnstoffe mit Alkoholen, Aminen oder Mercaptanen können herbizid wirksame Arylharnstoffe bzw. Carbamate oder Thiolcarbamate erhalten werden.

Die Halogenalkyl-phenylharnstoffe der Formel I lassen sich beispielsweise in an sich üblicher Weise mit Verbindungen der Formel

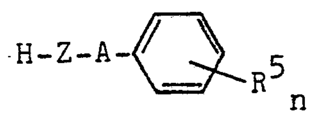

(V),

in der

Z Sauerstoff, Schwefel oder den Rest $-NR^6-$, wobei $R^6$ ein Alkyl mit 1 bis 5 Kohlenstoffatomen bedeutet,

A eine gegebenenfalls durch Alkyl mit 1 bis 5 Kohlenstoffatomen substituierte Alkylen- oder Alkenylenkette mit bis zu 5 Kohlenstoffatomen,

$R^5$ Wasserstoff, Halogen, Alkyl, Alkoxy, Halogenalkyl oder Halogenalkoxy mit jeweils bis zu 6 Kohlenstoffatomen, Phenyl oder Phenoxy und

n die Zahlen 1, 2 oder 3 bedeuten,

zu Anilinderivaten der Formel

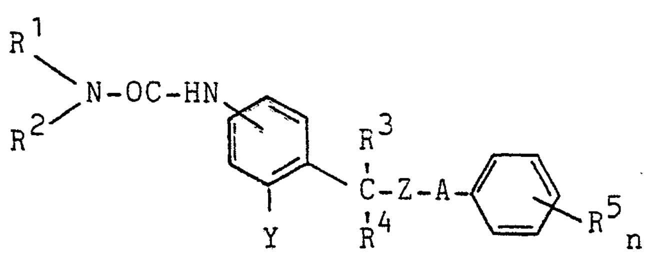

(VI),

in der $R^1$, $R^2$, $R^3$, $R^4$, $R^5$, A, Y, Z und n die oben genannten Bedeutungen haben und die Reste $-NH-CO-NR^1R^2$ und

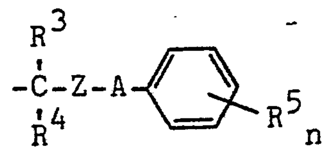

in m- oder p-Stellung stehen, umsetzen. Die Verbindungen der Formel VI sind herbizid wirksam.

Die Verbindungen der Formel I können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, z.B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstel-

lung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an festen

Trägerstoffen hergestellt werden. Feste Trägerstoffe sind Mineralerden wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten zwischen 0,1 und 95 Gewichtsprozent, vorzugsweise zwischen 0,5 und 90 Gewichtsprozent, Wirkstoff.

Beispiele für Formulierungen sind:

I. Man vermischt 90 Gewichtsteile der Verbindung Nr. 1 mit 10 Gewichtsteilen N-Methyl-alpha-pyrrolidon und erhält eine Lösung, die zur Anwendung in Form kleinster Tropfen geeignet ist.

II. 20 Gewichtsteile der Verbindung Nr. 2 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-mono-ethanolamid, 5 Gewichtsteilen Calciumsalz der Dodecyl-benzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gewichtsprozent des Wirkstoffs enthält.

III. 20 Gewichtsteile der Verbindung Nr. 1 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

IV. 20 Gewichtsteile der Verbindung Nr. 4 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

V. 20 Gewichtsteile der Verbindung Nr. 3 werden mit 3 Gewichtsteilen des Natriumsalzes der Diisobutylnaphthalin-alpha-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gewichtsprozent des Wirkstoffs enthält.

VI. 3 Gewichtsteile der Verbindung Nr. 5 werden mit 97 Gewichtsteilen feinteiligem Kaolin vermischt. Man erhält auf diese Weise ein Stäubemittel, das 3 Gewichtsprozent des Wirkstoffs enthält.

VII. 30 Gewichtsteile der Verbindung Nr. 45 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Teile der Verbindung Nr. 46 werden mit 2 Teilen Calciumsalz der Dodecylbenzolsulfonsäure, 8 Teilen Fettalkohol-polyglykolether, 2 Teilen Natriumsalz eines Phenol-Harnstoff-Formaldehyd-Kondensates und 68 Teilen eines paraffinischen Mineralöls innig vermischt. Man erhält eine stabile ölige Dispersion.

Die erfindungsgemäßen Verbindungen können im Vor- und Nachauflaufverfahren angewandt werden. Sind gewisse, bereits stehende Kulturpflanzen gegenüber den Wirkstoffen empfindlich, so können auch Ausbringungstechniken angewandt werden, bei welchen die herbiziden Mittel mit Hilfe der Spritzgeräte so geleitet werden, daß die Blätter empfindlicher Kulturpflanzen nach Möglichkeit nicht getroffen werden, während sie auf die unbedeckte Bodenfläche gelangen und die darin keimenden und aufwachsenden unerwünschten Pflanzen erreichen (post-directed, lay-by, Unterblattspritzung).

In Anbetracht der guten Verträglichkeit und der Vielseitigkeit der Applikationsmethoden und Formulierungsmöglichkeiten können die erfindungsgemäßen Herbizide in einer großen Zahl von Kulturpflanzen zur Beseitigung von unerwünschten Pflanzen eingesetzt werden. Die Aufwandmengen können dabei je nach Bekämpfungsvorhaben, Entwicklungsstadium der Pflanzen und Witterungsverhältnissen zwischen 0,1 und 15 kg/ha und mehr, vorzugsweise zwischen 1 bis 3 kg Wirkstoff/ha.

Die herbizide Wirkung der Halogen-alkyl-phenylharnstoffe der Formel I läßt sich durch Gewächshausversuche zeigen:

Als Kulturgefäße dienen Plastikblumentöpfe mit 300 $cm^3$ Inhalt und lehmigem Sand mit etwa 1,5 % Humus als Substrat. Die Samen der Testpflanzen werden nach Arten getrennt flach eingesät. Unmittelbar danach erfolgt bei Vorauflaufbehandlung das Aufbringen der Wirkstoffe auf die Erdoberfläche. Sie werden hierzu in Wasser als Verteilungsmittel suspendiert oder emulgiert und mittels fein verteilender Düsen gespritzt. Bei dieser Applikationsmethode beträgt die Aufwandmenge 2,0 kg Wirkstoff/ha. Nach dem Aufbringen der Mittel beregnet man die Gefäße leicht, um Keimung und Wachstum in Gang zu bringen. Danach deckt man die Gefäße mit durchsichtigen Plastikhauben ab, bis die Pflanzen angewachsen sind. Diese Abdeckung bewirkt ein gleichmäßiges Keimen der Testpflanzen, sofern dies nicht durch die Wirkstoffe beeinträchtigt wird.

Zum Zwecke der Nachauflaufbehandlung zieht man die Testpflanzen je nach Wuchsform erst bis zu einer Wuchshöhe von 3 bis 15 cm an und behandelt sie danach. Es werden entweder direkt gesäte und in den gleichen Gefäßen aufgewachsene Pflanzen ausgewählt, oder wie werden erst als Keimpflanzen getrennt angezogen und einige Tage vor der Behandlung in die Versuchsgefäße verpflanzt. Zur Aufzucht der Sojapflanzen setzt man der Topferde etwas Torfmull (peat) zu, um ein besseres Auflaufen und Wachsen zu gewährleisten. Die Aufwandmenge beträgt 2,0 bzw. 3,0 kg Wirkstoff/ha.

Die Versuchsgefäße werden im Gewächshaus aufgestellt, wobei für wärmeliebende Arten wärmere Bereiche (20 bis 35°C) und für solche gemäßigter Klimate 10 bis 25°C bevorzugt werden. Die Versuchsperiode erstreckt sich über 2 bis 4 Wochen. Während dieser Zeit werden die Pflanzen gepflegt, und ihre Reaktion auf die einzelnen Behandlungen wird ausgewertet. Bewertet wird nach einer Skala von 0 bis 100. Dabei bedeutet 100 kein Aufgang der Pflanzen bzw. völlige Zerstörung zumindest der oberirdischen Teile.

Die in den Gewächshausversuchen verwendeten Pflanzen setzen sich aus folgenden Arten zusammen:

Amaranthus retroflexus (zurückgekrümmter Fuchsschwanz), Centaurea cyanus (Kornblume), Chenopodium album (weißer Gänsefuß), Echinochloa crus-galli (Hühnerhirse), Glycine max. (Sojabohnen), Ipomoea spp. (Prunkwindearten), Triticum aestivum (Weizen), Sinapis alba (weißer Senf), Solanum nigrum (schwarzer Nachtschatten).

Bei der Prüfung auf herbizide Eigenschaften zeigt beispielsweise die Verbindung Nr. 1 eine selektive Wirkung bei Vorauflaufanwendung von 2,0 kg Wirkstoff/ha.

Im Nachauflaufverfahren bekämpfen beispielsweise die Verbindungen Nr. 3 und 46 mit 2,0 kg bzw. 3,0 kg Wirkstoff/ha unerwünschte Pflanzen.

Zur Verbreiterung des Wirkspektrums und zur Erzielung synergistischer Effekte können die neuen Verbindungen mit zahlreichen Vertretern anderer herbizider oder wachstumsregulierender Wirkstoffgruppen gemischt und gemeinsam ausgebracht werden. Beispielsweise kommen als Mischungspartner Diazine, 4 H-3,1-Benzoxazinderivate, Benzothiadiazinone, 2,6-Dinitroaniline, N-Phenylcarbamate, Thiolcarbamate,

Halogencarbonsäuren, Triazine, Amide, Harnstoffe, Diphenylether, Triazinone, Uracile, Benzofuranderivate, Cyclohexan-1,3-dionderivate und andere in Betracht.

Außerdem kann es von Nutzen sein, die neuen Verbindungen bzw. sie enthaltende herbizide Mittel allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- oder Spurenelementmängeln eingesetzt werden. Es können auch nicht-phytotoxische Öle und Ölkonzentrate zugesetzt werden.

Patentansprüche

1. Halogenalkylphenylharnstoffe der Formel

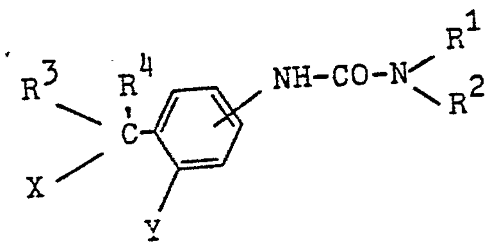 (I),

in der
$R^1$ und $R^2$ jeweils unabhängig voneinander Wasserstoff, Methoxy oder Methyl bedeuten,
Y Wasserstoff oder Chlor,
X Chlor oder Brom und
$R^3$ und $R^4$ jeweils unabhängig voneinander Wasserstoff oder Alkyl bedeuten,
mit der Maßgabe, daß $R^3$ und $R^4$ nicht gleichzeitig Wasserstoff bedeuten, wenn $R^1$ und $R^2$ für Methyl und Y für Wasserstoff stehen, und daß die Gruppe $-NH-CO-NR^1R^2$ nicht in o-Stellung zu Y oder zum Rest $-CXR^3R^4$ steht.

2. Verfahren zur Herstellung von Halogenalkylphenylharnstoffen der Formel I gemäß Anspruch 1, dadurch gekennzeichnet, daß man ein Halogenalkyl-phenylisocyanat der Formel

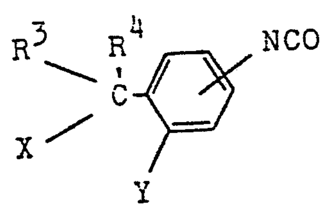 (II),

in der $R^3$, $R^4$ und Y die im Anspruch 1 genannten Bedeutungen haben, oder ein N-(Halogenalkylphenyl)-carbamoylhalogenid der Formel

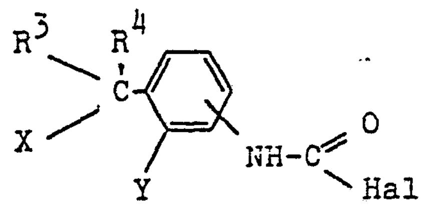

(III),

in der $R^3$, $R^4$ und Y die im Anspruch 1 genannten Bedeutungen haben und Hal für Halogen steht, in an sich üblicher Weise mit einem Amin der Formel

$HNR^1R^2$ (IV),

in der $R^1$ und $R^2$ die im Anspruch 1 genannten Bedeutungen haben, umsetzt.

3. Herbizid, enthaltend einen Halogenalkylphenylharnstoff der Formel I gemäß Anspruch 1.

4. Herbizid, enthaltend einen Halogenalkylphenylharnstoff der Formel

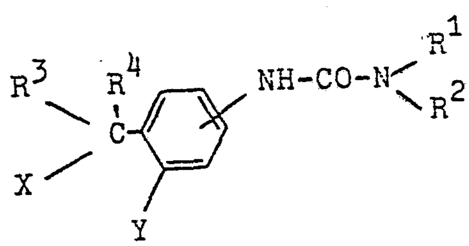

(I),

in der

$R^1$ und $R^2$ jeweils unabhängig voneinander Wasserstoff, Methoxy oder Methyl bedeuten,

Y Wasserstoff oder Chlor,

X Chlor oder Brom und

$R^3$ und $R^4$ jeweils unabhängig voneinander Wasserstoff oder Alkyl bedeuten,

mit der Maßgabe, daß die Gruppe $-NH-CO-NR^1R^2$ nicht in o-Stellung zu Y oder zum Rest $-CXR^3R^4$ steht.

5. Herbizid, enthaltend inerte Zusatzstoffe und einen Halogenalkylphenylharnstoff der Formel I gemäß Anspruch 4.

6. Verfahren zur Bekämpfung unerwünschten Pflanzenwuchses, dadurch gekennzeichnet, daß man die unerwünschten Pflanzen oder die von unerwünschtem Pflanzenwuchs freizuhaltende Fläche mit einer herbizid wirksamen Menge eines Halogenalkylphenyl-harnstoffs der Formel I gemäß Anspruch 1 behandelt.

7. Verfahren zur Bekämpfung unerwünschten Pflanzenwuchses, dadurch gekennzeichnet, daß man die unerwünschten Pflanzen oder die von unerwünschtem Pflanzenwuchs freizuhaltende Fläche mit einer herbizid wirksamen Menge eines Halogenalkylphenyl-harnstoffs der Formel I gemäß Anspruch 3 behandelt.

0091638

Europäisches Patentamt

# EUROPÄISCHER RECHERCHENBERICHT

Nummer der Anmeldung

EP 83 10 3303

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| X | DE-A-2 909 828 (SUMITOMO CHEMICAL CO.) * Seite 32, Zeile 15; Seite 33, Zeile 23 * | 1 | C 07 C 127/19<br>A 01 N 47/30 |
| | --- | | |
| A | DE-A-2 501 729 (CELAMERCK GmbH) * Seite 7, Ansprüche 1-3 * | 1-3 | |
| | --- | | |
| A | DE-A-2 503 736 (BAYER AG.)<br>* Seite 14, Beispiel 2 - Seite 16, Beispiel 5 * | | |
| | ----- | | |
| | | | RECHERCHIERTE SACHGEBIETE (Int. Cl. 3) |
| | | | C 07 C 127/00 |

Der vorliegende Recherchenbericht wurde für alle Patentansprüche erstellt.

| Recherchenort | Abschlußdatum der Recherche | Prüfer |
|---|---|---|
| DEN HAAG | 04-08-1983 | SUTER M. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82